# EUROPEAN PATENT SPECIFICATION

(11) **EP 0 743 052 B1**
(45) Date of publication and mention of the grant of the patent: **02.11.2000**
(21) Application number: 96113342.8
(22) Date of filing: 09.12.1992
(51) Int. Cl.: A61F 13/15

(54) **Disposable three-dimensional garment**
Dreidimensionales Wegwerfkleidungsstück
Vêtement jetable tridimensionnel

(30) Priority: 18.12.1991 US 809993
(43) Date of publication of application: 20.11.1996
(62) Divisional of application: 92121010.0
(73) Proprietor: KIMBERLY-CLARK WORLDWIDE, INC., Neenah, Wisconsin 54956 (US)
(72) Inventor: Van Gompel, Paul Theodore, Hortonville, WI 54944 (US); Suprise, Jody Dorothy, Neenah, WI 54956 (US); Schleinz, Robert Joseph, Appleton, WI 54915 (US); Ziegler, Diane Mildred, Appleton, WI 54914 (US); Popp, Robert Lee, Hortonville, WI 54944 (US); Poklasny, June Marjorie, Oshkosh, WI 54901 (US)
(74) Representative: Diehl, Hermann, Dr. Dipl.-Phys.

(56) References cited:
- EP-A- 0 323 040
- EP-A- 0 323 634
- FR-A- 2 541 872
- US-A- 4 646 362
- US-A- 4 701 171
- US-A- 4 854 985

## Description

This invention pertains to absorbent garments, and more particularly to a disposable three-dimensional garment,

When a child reaches an age in the range of about 15 to 30 months, the parent or parents generally desire to start toilet training the child so he or she can become independent of the parent. The training pant is intended for use when the child has reached an age at which he or she is ready to graduate to three-dimensional garments as a replacement for two-dimensional diapers previously used. Thus, a suitable training pant has closed sides and permits a child to raise and lower it easily along the legs without requiring the aid of a parent. At the same time, a training pant must provide features of liquid and solid absorbency and the prevention of waste leakage.

One example of a training pant is the child's training pant in U.S. Pat. No. 4,646,362, assigned to the assignee of the present invention. This patent describes a training pant having a two-ply outer cover, a body side liner, and an absorbent therebetween. The training pant has an elasticized waist opening and a pair of elasticized leg openings.

The training pant according to this document does not comprise an inner layer of heat shrinkable material being heat treated at least at respective portions thereof defining the pair of side sections, whereby said side sections are made stretchable.

The US-A-4 854 985 discloses a method for manufacture of elastic leg and waist disposable diapers and the products produced thereby. The moisture impermeable backing sheet of this diaper is formed of an elastomeric material which is heat unstable and relatively inelastic in its unshrunk form, and stable and relatively elastic in its heat shrunk form. Alternatively, a conventional polyolefin backing sheet may be used with strips of heat shrinkable elastomeric material bonded in appropriate marginal locations in the waist or leg zones. The resulting diapers have elastic leg and waist areas. No elastic zones are provided in side sections of the diaper and the outer cover of this diaper is neither a full outer cover nor is it a two-layered outer cover.

Another example of a training pant is that in U.S. Pat. No. 4,940,464, which is assigned to the assignee of the present invention. This training pant is different from that described in U.S. Pat. No. 4,646,362 in that it has stretchable side panels that maintain the central absorbent assembly snugly against the child's body.

Besides children, adults experience incontinence problems and some adults use several types of garments to hide or provide for their incontinence. For example, adults who experience incontinence generally try to hide their problem by use of feminine pads, diapers, or larger diaper-like articles that are sized to fit the adult anatomy. These adults have need of a satisfactory three-dimensional garment to care for their needs. Also, women's menstrual needs need to be provided for with improved pant-like garments that can be discreetly worn with different types of clothing.

It is therefore an object of the present invention to provide an improved disposable three-dimensional garment, such as a training pant or a garment to hide adult incontinence. This object is solved by the disposable three dimensional garment of independent claim 1.

Further advantageous features, aspects and details of the invention are evident from the dependent claims, the description and the drawings. The claims are intended to be understood as a first nonlimiting approach of defining the invention in general terms.

According to the present invention, a disposable absorbent three-dimensional garment is provided, said garment comprising a two-layered full outer cover comprising an inner layer of heat-shrinkable material and an outer layer of nonwoven material, said two-layered full outer cover defining a front section, a back section, a crotch section, a pair of side sections, a waist opening and a pair of leg openings, an absorbent medium at said crotch section and a stretchable waistband joined to said waist opening, said inner layer of heat-shrinkable material of said two-layered full outer cover being heat-treated at least at respective portions thereof defining said pair of side sections, whereby said side sections are made stretchable.

According to one aspect there is provided a disposable three-dimensional absorbent garment comprising a full outer cover including an inner surface, an outer surface, a pair of side sections and waist and leg openings. A pair of stretchable side members is provided on the inner surface of the outer cover at respective side sections to provide stretch to the outer cover. An absorbent medium is provided at the crotch section of the outer cover.

According to another aspect there is provided a disposable three-dimensional absorbent garment comprising an outer cover having an inner surface, an outer surface, a pair of side sections, and waist and leg openings. Visual characters may be provided on the outer cover. A pair of stretchable side members are operatively joined with the inner surface at respective side sections, and each stretchable side member includes a stretch gradient between the waist opening and a leg opening. There is an absorbent at the crotch section of the outer cover.

According to yet another aspect, a disposable three-dimensional absorbent garment is provided comprising a full outer cover having an inner surface, an outer surface, a pair of side sections, and waist and leg openings. A pair of triangularly-shaped stretchable side members are joined with the inner surface at the side sections. A stretchable waistband is joined to the waist opening. A vertex of each stretchable side member is positioned generally adjacent the waistband. An absorbent medium is provided at the crotch section of the outer cover.

Furthermore, a method of making a disposable three-dimensional absorbent garment is disclosed, said method comprising the steps of providing an outer cover having an inner surface, an outer surface, and opposite lateral sides; operatively joining a pair of stretchable side members with the inner surface of the outer cover; attaching portions of the lateral sides together to form a waist opening, a pair of leg openings, and a pair of stretchable side sections that include the stretchable side members; and providing an absorbent medium on the outer cover.

The above-mentioned and other features and aspects of this invention will become more apparent and the invention itself will be better understood by reference to the following description of the examples of the invention, taken in conjunction with the accompanying drawings, wherein:
Fig. 1 is a perspective view of a preferred embodiment of the present invention;
Fig. 2 is a perspective view of a modification of the embodiment in Fig. 1 without visual characters;
Fig. 3 is a perspective view of another modification of the embodiment in Fig. 1 without visual characters;
Fig. 4 is a top plan view of the embodiment of Fig. 1 with the seams unjoined and the embodiment laid flat with the liner partially broken away to expose interior elements;
Fig. 5 is a view similar to Fig. 4 of the modification in Fig. 2;
Fig. 6 is a broken-away view similar to Fig. 4 of the modification in Fig. 3;
Fig. 7 is a cross-sectional view illustrating one arrangement of various elements;
Fig. 8 is a top plan view illustrating one process of attaching stretchable members; and
Fig. 9 is similar to Fig. 8 with the stretchable members in a relaxed, joined state.

### Definitions

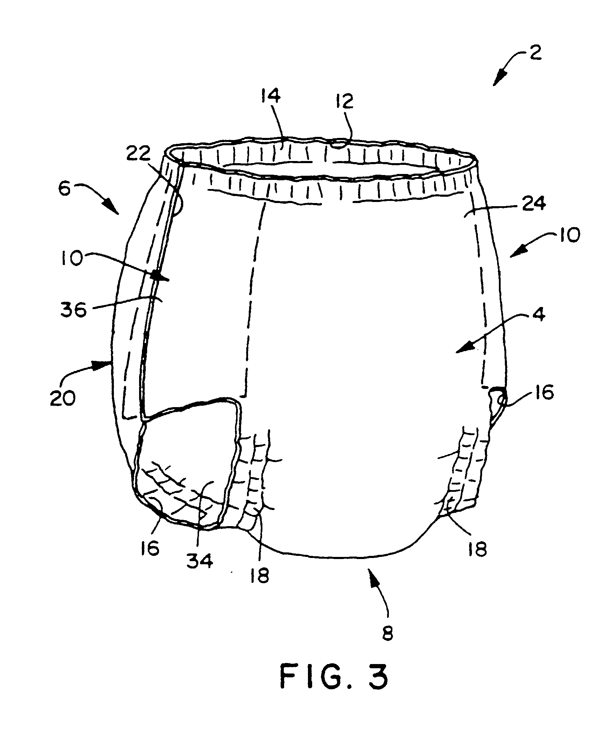

Within the context of this specification, each term or phrase below will include the following meaning or meanings:
(a) "SBL" means a stretch bonded laminate that is at least a two-layered composite in which one layer is a gatherable layer and the other layer a stretchable layer. The layers are joined together when the stretchable layer is in a stretched condition so that upon relaxing the layers, the gatherable layer is gathered.
(b) "Two-dimensional" refers to a garment that can be opened without tearing any structure and laid in a flat condition. These garments, such as diapers, do not have continuous leg and waist openings, and require a fastening device, such as tapes, to attach about the wearer.
(c) "Three-dimensional" refers to a garment similar to shorts or pants in that they have continuous leg and waist openings that are bounded by material of which the garment is made.
(d) "Full outer cover" means that the outer cover is one piece or unitary and defines the form of a three-dimensional pant.
(e) "Horizontal" or "horizontally" refers to a left-to-right and/or right-to-left direction as viewed in the Figures, particularly with reference to Fig. 3, and does not require an orientation congruent with the true horizontal. An element that is horizontally disposed or oriented includes that element oriented in the true horizontal or within about 45 degrees of true horizontal.
(f) "Member" when used in the singular can have the dual meaning of a single panel-like member, or a plurality of bands or strings.
(g) "Operatively joined" with reference to the attachment of a stretchable member to another element means that the stretchable member when attached to or connected to or treated with heat with the element makes that element stretchable. The joining can be either directly, such as joining the stretchable member directly on the element, or can be joined indirectly by means of a second element disposed between the stretchable member and the first element.
(h) "Triangularly shaped" includes not only a true triangle, but also a truncated triangle. The sides may be linear or curvilinear. The shape is three-sided.
(i) "Stretch", "stretchability", "stretch characteristics", and variations thereof can be used interchangeably and mean that the material which is being described can be stretched and upon relaxing will tend to resume its original shape. The force necessary to stretch the material is the tension of or on the stretched material.

These definitions are not intended to be limiting, and these terms may be defined with additional language in the remaining portion of the specification.

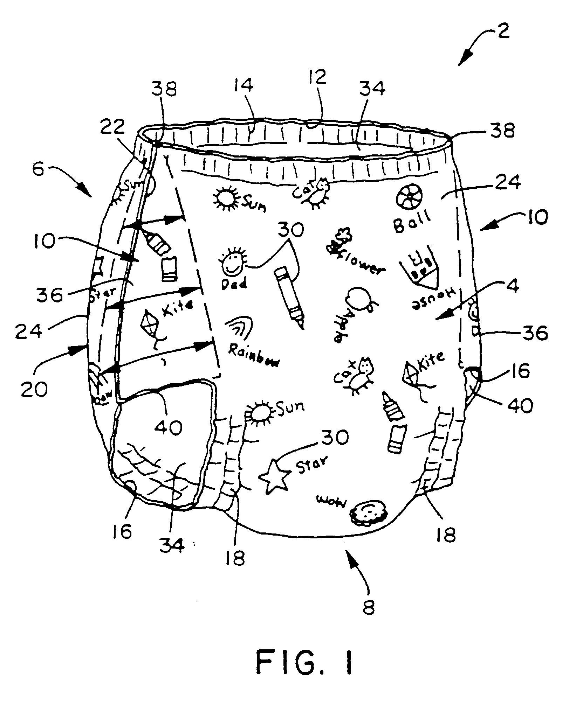

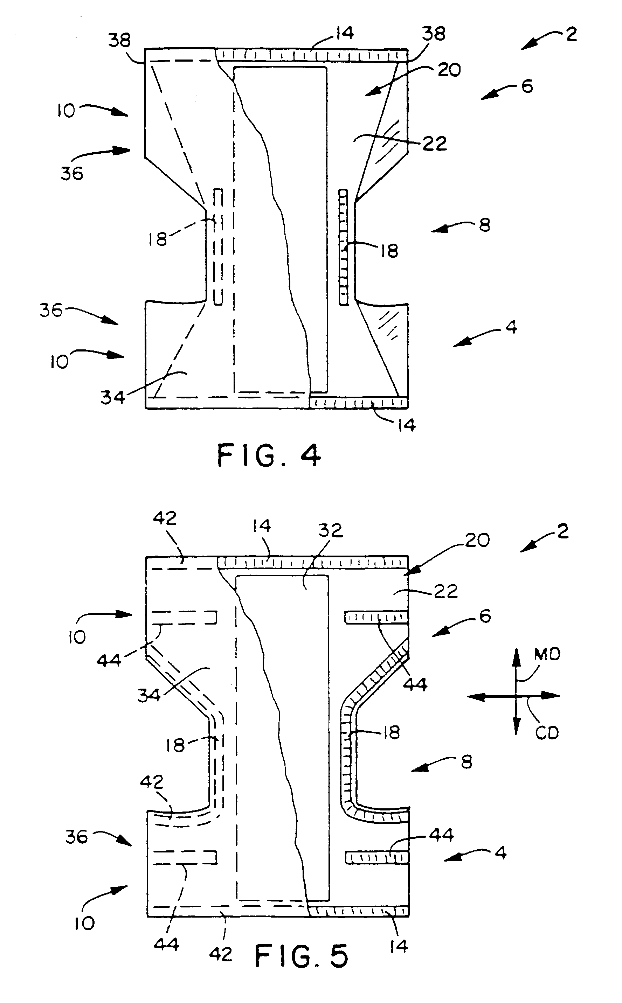

Although the current invention is contemplated as being used by adults or by children as a child's training pant, the following description will focus solely on a child's training pant for ease in describing and understanding the invention. Referring primarily to Figs. 1 and 4, disposable three-dimensional training pant 2 comprises in major part front section 4, back section 6, crotch section 8, a pair of side sections 10, waist opening 12 having a stretchable waistband 14, and a pair of leg openings 16 having respective stretchable leg bands 18.

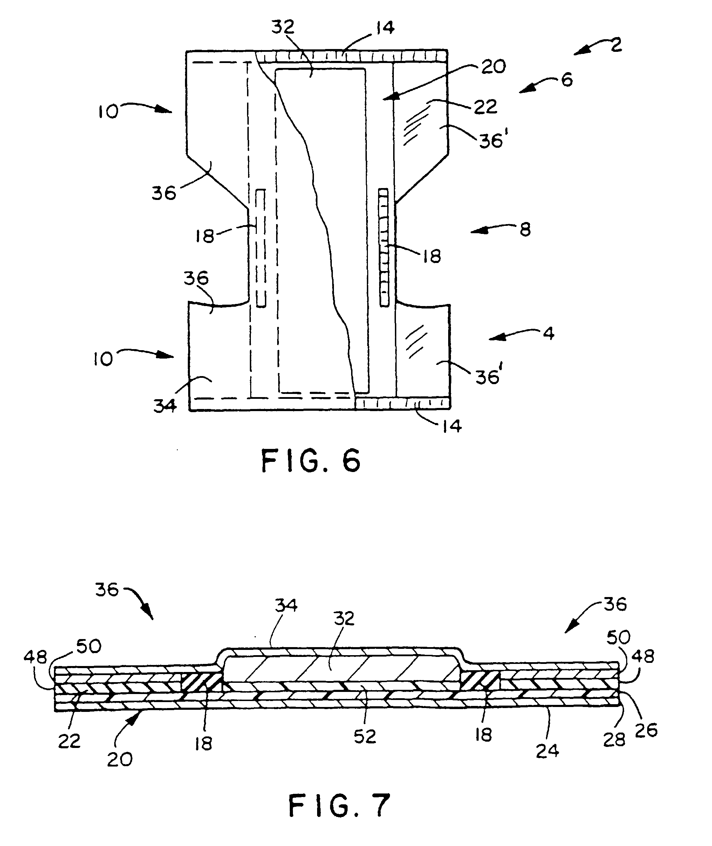

Referring also to Fig. 7 along with Figs. 1 and 4, training pant 2 further comprises a full outer cover 20 having an inner surface 22 and an outer surface 24. Outer cover 20 is preferably liquid impermeable, and can comprise an inner layer 26 of a liquid impermeable material joined to an outer layer 28 of nonwoven material. This type of two-layered outer cover is more specifically described in U.S. Pat. No. 4,646,362, which is incorporated by reference herein. When outer cover 20 is a two-ply laminate with inner layer 26 and outer layer 28, then inner surface 22 is that surface of inner layer 26 that faces inwardly toward the wearer, and outer surface 24 is that surface of outer layer 28 that faces outwardly away from the wearer. Outer cover 20 can also be liquid permeable and made of a non-woven material such as the material of which outer layer 28 can be made. If desired, outer cover 20 can be liquid impermeable and vapor permeable.

Outer cover 20 also includes a plurality of visual characters 30 on its outer surface 24. These characters 30 are not necessary for the waste containment performance of the invention and may be eliminated if desired. These characters 30 can include any type or number and combination of animals, letters, objects, toys, scenes, and the like which may be of interest to and/or educational for the child. If outer cover 20 is a single ply outer cover, then visual characters 30 are printed, or in any other suitable manner formed, on outer surface 24. However, if outer cover 20 includes inner layer 26 and outer layer 28, then visual characters 30 can be on inner layer 26 such that they are sandwiched between inner layer 26 and outer layer 28, or can be on the surface of outer layer 28 that is opposite from inner layer 26.

Training pant 2 further comprises absorbent medium 32 fixed between outer cover 20 and body side liner 34 in any suitable manner known in the art.

Training pant 2 further includes a pair of stretchable side members 36 operatively joined with inner surface 22 of outer cover 20 to make side sections 10 stretchable. By being operatively joined to inner surface 22, all of outer surface 24 of outer cover 20 is totally or fully exposed to view, thereby providing a more pant-like appearance. The pant-like appearance is further enhanced by visual characters 30 being included at side sections 10, as well as front section 4, back section 6, and crotch section 8.

Continuing to refer to Figs. 1 and 4, each stretchable side member 36 includes a vertex 38 that is generally adjacent waist opening 12 and a base 40 generally adjacent a respective leg opening 16. Stretchable side members 36 are preferably disposed between outer cover 20 and liner 34, and preferably attached to both outer cover 20 and liner 34. As illustrated in Fig. 1, it can be appreciated that the triangularly-shaped design of stretchable side members 36 provides a stretch-gradient to respective side sections 10, in which the amount of stretchability increases from waist opening 12 to a respective leg opening 16 as illustrated by the horizontal arrows within stretchable side member 36 of Fig. 1. Within the context of this description, as a stretchable material is increasingly stretched, the force applied by that material to or against or upon the wearer also increases. By providing this type of stretch gradient to side sections 10, training pant 2 of the present invention has the preferred characteristics of being easily pulled up the legs to the waist by the wearer, while providing a snug fit against the wearer in order to maintain absorbent medium 32 snugly in place to absorb waste material. Because stretchability is provided about the total periphery of waist opening 12 by waistband 14, it is easier to open or expand as compared to a waist opening that is only partially stretchable. In addition, absorbent medium 32 is maintained snugly in place by leg bands 18 and stretchable side members 36, thereby providing the desired waste containment.

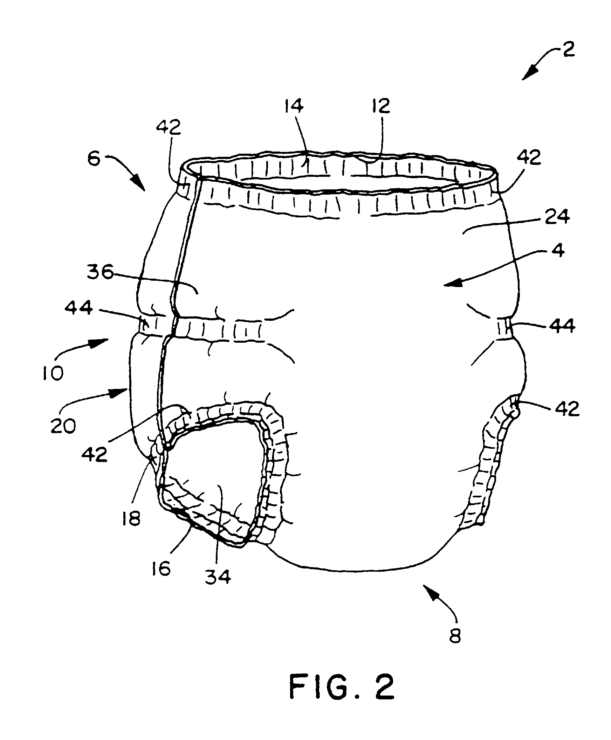

Referring now to Figs. 2 and 5, training pant 2 has been modified by substituting the generally triangularly-shaped stretchable side member 36 in each side section 10 by a plurality of generally horizontally disposed stretchable bands 42, 44. Fig. 2 illustrates these stretchable bands 42, 44 in side section 10 as including that portion of stretchable waistband 14 in side section 10, that portion of stretchable leg band 18 in side section 10, and an intermediate stretchable band 44. Naturally, a fewer number or greater number of bands 42, 44 can be provided, and may also take the shape of strings or slender thread-like filaments. The stretch gradient can be provided in several ways. For example, the stretchable materials of which waistband 14, intermediate stretchable band 44, and stretchable leg band 18 are made can vary in stretchability or cross-sectional area such that the stretchability of each side section 10 increases from the waist opening 12 to a respective leg opening 16.

A stretch gradient can also be provided for each stretchable side band 42, 44 in Fig. 2 by using the same type of stretchable material and selectively treating the material. For example, stretchable leg band 18 would not receive any selective treatment, whereas intermediate stretchable band 44 could receive a first amount of selective treatment, and that portion of stretchable waistband 14 in side section 10 could receive a second selective treatment greater than the first treatment of band 44, in which the treatments minimize or decrease the stretchability. A stretch gradient can also be provided by using different types of stretchable materials. For example, waistband 14 would be made of a material having one stretchability, intermediate stretchable band 44 could be made of another material having a second stretchability greater than waistband 14, and leg band 18 could be made of a material having a third stretchability greater than intermediate stretchable band 14, thereby providing increased stretchability from the waist opening 12 to a respective leg opening 16.
As illustrated in Fig. 5, waistband 14, intermediate band 44, and leg bands 18 are preferably disposed between outer cover 20 and liner 34, and preferably attached to both outer cover 20 and liner 34.

Referring now to Figs. 3 and 6, another modification to training pant 2 illustrated in Fig. 1 has generally rectangularly-shaped side members 36'. In order to provide the desired stretch gradient in which the stretchability increases in a direction from waist opening 12 to leg openings 16, selected portions of each rectangularly-shaped stretchable side member 36' can be selectively treated or modified. For example, a plurality of bond points, which would not cause holes through each side member 36', could be provided in increasing concentration or density in a direction from a respective leg opening 16 to waist opening 12, thereby providing a stretch gradient in which the stretchability increases in a direction from waist opening 12 to a respective leg opening 16. Alternatively, a plurality of slits or openings (not shown) may also be provided through each side member 36' in increasing concentration or density in a direction from waist opening 12 to a respective leg opening 16.

The present invention contemplates other structures and designs that result in a stretch gradient between the waist opening 12 and respective leg openings 16, and the above descriptions are only representative. Although the above description has described the preferred stretch gradient as having stretchability that increases in a direction from waist opening 12 to a respective leg opening 16, the present invention also contemplates the reverse, if desired, in which the stretchability would decrease from waist opening 12 to a respective leg opening 16. The stretch gradient also can increase-decrease-increase or decrease-increase-decrease between waist opening 12 and a respective leg opening 16. In other words, an intermediate portion of the stretch gradient can have greater or lesser stretchability than end portions thereof.

With reference to Fig. 7, stretchable side member 36 can be a two-layered composite. As described above, this composite can be made with several different processes and materials and configurations. One composite and process includes a heat-shrinkable layer 48 combined or associated in any suitable manner with any suitable nonwoven layer 50. This nonwoven layer 50 is preferably a polyester powder-bonded carded web. The composite can then be cut into any suitable shape for forming stretchable side members 36, for example into a triangular shape, rectangular shape, or bands or strings. Thereafter, stretchable side member 36 is placed on outer cover 20 such that layer 48 is contacting inner surface 22 of a side section 10. Layer 48 can then be thermally bonded to inner layer 22 and liner 34, thereby causing layer 48 to be stretchable. Suitable heat-shrinkable materials can be purchased from the Exxon Chemical Company.

Another process includes a 2-layer outer cover 20 in which the inner layer, such as liquid impermeable inner layer 26, is a heat-shrinkable material. Side sections 10 are made stretchable by application of heat as required for the heat-shrinkable material used.

Another process includes forming a composite into a desired shape for use as a stretchable side member 36. This composite can be a 2-layered composite in which one layer is a meltblown elastomer having a basis weight between about 40 to about 150 grams per square meter (gsm) and which is formed onto a second layer of a nonwoven material having a basis weight between about 5 to about 30 gsm. This formed composite can then be cut into any desired shape. Each formed and cut composite is then bonded, such as by thermal bonding, to inner surface 22 of outer cover 20 with the elastomer being sandwiched by the outer cover and the second layer of nonwoven material. In this case, outer cover 20 can be a nonwoven material having a basis weight between about 20 to about 60 gsm. Outer cover 20 can then be passed through a series of cross-directional softening rolls that cause only the composite to become stretchable in the horizontal or cross-direction, thereby providing stretchable side members 36.

Another process includes attaching a stretch bonded laminate material while in a stretched condition to inner surface 22 of side sections 10 and liner 34.

With reference to Fig. 7, outer cover 20 can be a single layer of nonwoven material having a basis weight of about 20 to about 60 gsm, and which is liquid permeable. In this case, a liquid impermeable layer 52 is provided between absorbent 32 and outer cover 20. However, liquid impermeable layer 52 can be eliminated in the case in which outer cover 20 comprises liquid impermeable inner layer 26 and outer layer 28.

Generally, waistband 14 will be a heat-shrinkable material preferably joined between outer cover 20 and liner 34, and thereafter selectively heated. In the case of leg bands 18, they are generally applied in a stretched condition before being preferably joined between outer cover 20 and liner 34.

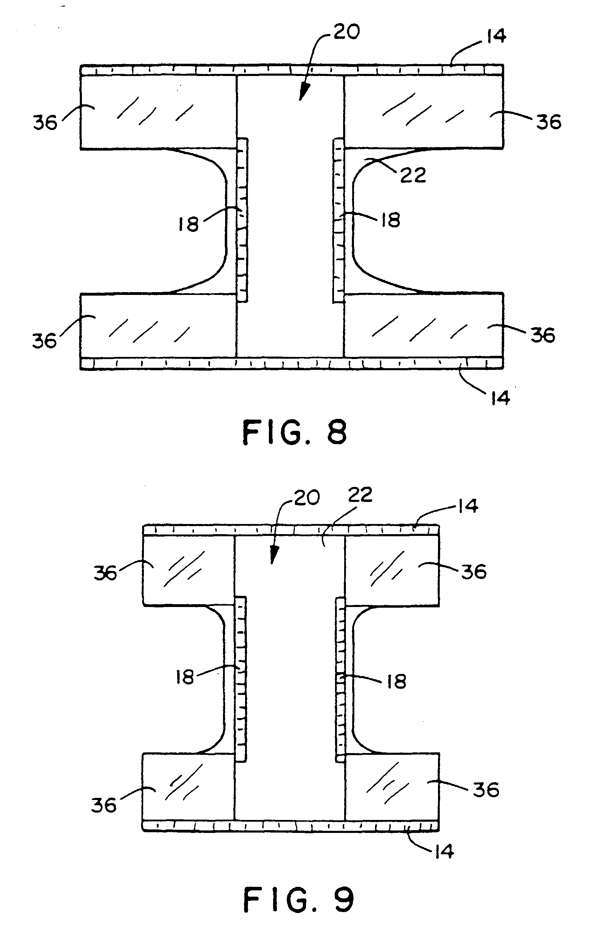

Referring now to Figs. 8 and 9, there is illustrated another method of joining stretchable side members 36 to outer cover 20. In this method, outer cover 20 and liner 34 are presized larger than the end-product size. The amount of presizing is naturally dependent upon the type of stretchable side member joined thereto. As illustrated in Fig. 8, stretchable waistband 14 and stretchable side members 36 are stretched in the cross-direction and then joined to inner surface 22 and liner 34 (not shown), while leg bands 18 are stretched in the machine direction and joined to inner surface 22 and liner 34 (not shown). Thereafter, waistband 14, stretchable side members 36, and leg bands 18 are allowed to relax, thereby gathering or shrinking outer cover 20 and liner 34 to the desired end-product size. Absorbent medium 32 is positioned between outer cover 20 and liner 34 during the placement of the stretchable members, and attached to outer cover 20 by lines of adhesive.
As earlier described, outer cover 20 can be a two-ply layer as described in U.S. Pat. No. 4,646,362. However, outer cover 20 may also be a single layer of any suitable material that is liquid impermeable. Examples include meltblown or film material made of polypropylene or polyolefin, copolymers such as ethylene vinyl acetate, ethylene methyl acrylate, ethylene ethyl acrylate, polyvinyl chloride, and the like. Other materials include a single spunbonded layer of the above types of material suitably treated or coated to be liquid impermeable; two layers of spunbonded and meltblown materials suitably treated or coated to be liquid impermeable; or three layers of material of spunbonded-meltblown-spunbonded material suitably treated or coated to be liquid impermeable. Outer cover 20 may also be made of a material that is liquid impermeable, and vapor permeable. In the case in which liquid impermeable layer 52 is used as illustrated in Fig. 7, then outer cover 20 may be made of any material of which liner 34 is made.

Liner 34 may be a liquid permeable, hydrophilic or hydrophobic material, such as a spunbonded web composed of synthetic polymer filaments; a spunlace web; a spunbond-meltblown web; a meltblown web; or a bonded carded web composed of synthetic polymer fibers. Suitable synthetic polymers include polyethylene, polypropylene, polyester, and nylon.

Stretchable side members 36 can be made of any suitable materials, such as those of which the stretchable side members of U.S. Pat. No. 4,940,464 are made. Similarly, waistband 14 and leg bands 18 can be made of any suitable materials, such as those of which the waistband and leg bands of U.S. Pat. No. 4,940,464 are made.

Absorbent medium 32 may be made of any suitable absorbent material such as cellulosic fibers, synthetic fibers, absorbent gelling materials in the form of particles, fibers, layers, and the like. These various materials may be combined as mixtures or blends. They also may be discrete layers such as a discrete layer of cellulosic fiber and a discrete layer of absorbent gelling material, or a coform-type layer which is a blend or mixture of synthetic and cellulosic fibers formed as a coform layer with a discrete layer of absorbent gelling materials placed therewith. Suitable absorbent gelling materials can be inorganic materials such as silica gels or organic compounds such as cross-linked polymers. Examples include polyacrylamides, polyvinyl alcohol, polyacrylates, acrylonitrile grafted starch, acrylic acid grafted starch, and the like. Absorbent medium 32 can also include a tissue wrap to maintain the integrity thereof.

Based on the foregoing description, several embodiments of three-dimensional absorbent garments are described. One suitable disposable three-dimensional absorbent garment (2) may comprise a full outer cover (20) comprising an inner surface (22), an outer surface (24), a pair of side sections (10), and waist (12) and leg openings (16), a pair of stretchable side members (36,36'), one of said stretchable side members (36,36',42,44) being operatively joined with said inner surface (22) of said full outer cover (20) at one of said side sections (10), the other of said stretchable side members (36,36',42,44) being operatively joined with said inner surface at the other of said side sections (10), and an absorbent medium (32) at a crotch section (8) of said full outer cover.

Another suitable disposable three-dimensional absorbent garment (2) could comprise an outer cover (20) comprising an inner surface (22), an outer surface (24), a pair of side sections (10), and waist (12) and leg openings (16), a pair of stretchable side members (36,36',42,44) being operatively joined with said inner surface (22) of said outer cover (20) at respective ones of said side sections (10), each said stretchable side member (36,36',42,44) including a stretch gradient between said waist opening (12) and a respective said leg opening (16), and an absorbent medium (32) at a crotch section (8) of said outer cover (20).

Preferably, said outer cover (20) is a full outer cover.

The garment may further comprise visual characters (30) on said outer cover (20).

Furthermore, said outer cover (20) could include an inner layer (26) and an outer layer (28), said visual characters (30) being between said inner and outer layers or, alternatively, on said outer layer.

The stretchable side members (36,36',42,44) may include a stretch gradient at respective said side sections between said waist opening (12) and said leg openings (16), whereby said stretch gradient of each said stretchable side member (36,36',42,44) could increase in a direction from said waist opening (12) to a respective said leg opening (16). Otherwise, said stretch gradient of each said stretchable side member (36,36',42,44) could decrease in a direction from said waist opening (12) to a respective said leg opening (16).

Preferably, said stretch gradient between said waist opening (12) and a respective said leg opening (16) varies in stretchability such that an intermediate portion of said stretch gradient is more stretchable than end portions thereof.

Alternatively, said stretch gradient between said waist opening (12) and a respective said leg opening (16) varies in stretchability such that an intermediate portion of said stretch gradient is less stretchable than end portions thereof.

Preferably, each of said stretchable side members (36) is generally triangularly shaped or rectangularly shaped.

According to a further aspect, each said stretchable side member comprises a plurality of spaced-apart stretchable members (42,44).

A stretchable waistband (14) may be operatively joined to said waist opening (12) and/or a stretchable leg band (18) may be operatively joined to a respective said leg opening (16).

Advantageously, a pair of triangularly-shaped stretchable side members (36) is operatively joined with said inner surface (22) of said outer cover (20) at respective ones of said side sections (10), a vertex of each said triangularly-shaped stretchable side members (36) being generally adjacent said waist opening (12).

According to a further aspect there is disclosed a disposable three-dimensional absorbent garment (2) comprising a full outer cover (20) comprising an inner surface (22), an outer surface (24), a pair of side sections (10), and waist (12) and leg openings (16), a pair of triangularly-shaped stretchable side members (36) being operatively joined with said inner surface (22) of said full outer cover (20) at respective ones of said side sections (10), a stretchable waistband (14) operatively joined to said waist opening (12), a vertex of each said triangularly-shaped stretchable side members (36) being generally adjacent said stretchable waistband (14) and an absorbent medium (32) at a crotch section (8) of said full outer cover (20).

Again, visual characters (30) may be provided on said outer cover (20).

A stretchable leg band (18) can be operatively joined to a respective said leg opening (16).

The aforementioned garments could be manufactured by a method of making a disposable three-dimensional absorbent garment, said method comprising the steps of providing an outer cover having an inner surface, an outer surface and opposite lateral sides; operatively joining a pair of stretchable side members with the inner surface of the outer cover; attaching selected portions of the lateral sides together to form a waist opening, a pair of leg openings, a pair of stretchable side sections that include the stretchable side members and a crotch section that includes the absorbent medium; and providing an absorbent medium on the outer cover.

Additionally, said method further comprises the step of providing visual characters on the outer cover.

Preferably, the step of providing an outer cover further includes providing the outer cover with an inner layer and an outer layer and the step of providing visual characters includes providing the visual characters between the inner and outer layers.

Alternatively, the step of providing an outer cover further includes providing the outer cover with an inner layer and an outer layer and the step of providing visual characters includes providing the visual characters on the outer layer.

Advantageously, the step of operatively joining the stretchable side members further includes the step of providing stretch gradients to the respective stretchable side members such that the formed side sections include the stretch gradients.

Additionally, said method may further comprise the step of operatively joining a stretchable waistband to the outer cover and a pair of stretchable leg bands to the outer cover.

## Claims

1. A disposable absorbent three-dimensional garment (2) comprising:
a two-layered outer cover (20) comprising an inner layer of heat-shrinkable material and an outer layer of nonwoven material,
said two-layered full outer cover (20) defining a front section (4), a back section (6), a crotch section (8), a pair of side sections (10), a waist opening (12) and a pair of leg openings (16),
an absorbent medium (32) at said crotch section (8), and
a stretchable waistband (14) joined to said waist opening (12),
characterized in that
the outer cover is a full outer cover and in that said inner layer of heat-shrinkable material of said two-layered full outer cover (20) being heat-treated at least at respective portions thereof defining said pair of side sections (10), whereby said side sections (10) are made stretchable.

2. The garment of claim 1 wherein said side sections (10) have respective stretch gradients.

3. The garment of claim 1 or 2 further comprising visual characters on said outer cover (20).

4. The garment of claim 3 wherein said visual characters are between said inner layer and said outer layer.

5. The garment of claim 3 or 4 wherein said visual characters are on an outer surface of said outer cover (20).

## Patentansprüche

1. Saugfähiges, dreidimensionales Einwegkleidungsstück (2), das umfasst:
eine zweischichtige äußere Hülle (20), die eine innere Schicht aus einem wärme-schrumpffähigen Material und eine äußere Schicht aus einem Vliesmaterial umfasst,
wobei die zweischichtige vollständige äußere Hülle (20) einen Vorderabschnitt (4), einen Rückabschnitt (6), einen Schrittabschnitt (8), ein Paar Seitenabschnitte (10), eine Taillenöffnung (12) und ein Paar Beinöffnungen (16) abgrenzt,
ein saugfähiges Mittel (32) im Schrittabschnitt (8) und
einen dehnbaren Taillenbund (14), der mit der Taillenöffnung (12) verbunden ist,
dadurch gekennzeichnet, daß
die äußere Hülle eine vollständige äußere Hülle ist und daß die innere Schicht aus wärme-schrumpffähigem Material der zweischichtigen vollständigen äußeren Hülle (20) zumindest an jeweiligen Abschnitten davon, die das Paar Seitenabschnitte (10) abgrenzen, wärmebehandelt ist, wobei die Seitenabschnitte (10) dehnbar ausgelegt sind.

2. Kleidungsstück nach Anspruch 1, wobei die Seitenabschnitte (10) jeweilige Dehngradienten aufweisen.

3. Kleidungsstück nach Anspruch 1 oder 2, das des weiteren visuelle Zeichen auf der äußeren Hülle (20) aufweist.

4. Kleidungsstück nach Anspruch 3, wobei sich die visuellen Zeichen zwischen der inneren Schicht und der äußeren Schicht befinden.

5. Kleidungsstück nach Anspruch 3 oder 4, wobei sich die visuellen Zeichen auf einer äußeren Fläche der äußeren Hülle (20) befinden.

## Revendications

1. Vêtement tridimensionnel absorbant jetable (2) comprenant :
une enveloppe extérieure à deux couches (20) comprenant une couche intérieure d'un matériau thermo-rétrécissable et une couche extérieure d'un matériau non tissé,
ladite enveloppe extérieure totale à deux couches (20) définissant une section avant (4), une section arrière (6), une section d'entrejambe (8), une paire de sections latérales (10), une ouverture de ceinture (12) et une paire d'ouvertures de jambe (16),
un milieu absorbant (32) au niveau de ladite section d'entrejambe (8), et
une ceinture extensible (14) réunie à ladite ouverture de ceinture (12), caractérisé en ce que
l'enveloppe extérieure est une enveloppe extérieure totale et en ce que ladite couche intérieure de matériau thermo-rétrécissable de ladite enveloppe extérieure totale à deux couches (20) est traitée thermiquement au moins dans des portions respectives de celle-ci définissant ladite paire de sections latérales (10), grâce à quoi lesdites sections latérales (10) sont rendues extensibles.

2. Vêtement selon la revendication 1, dans lequel lesdites sections latérales (10) ont des gradients d'extensibilité respectifs.

3. Vêtement selon la revendication 1 ou 2, comprenant en outre des caractères visuels sur ladite enveloppe extérieure (20).

4. Vêtement selon la revendication 3, dans lequel lesdits caractères visuels sont situés entre ladite couche intérieure et ladite couche extérieure.

5. Vêtement selon la revendication 3 ou 4, dans lequel lesdits caractères visuels sont sur une surface extérieure de ladite enveloppe extérieure (20).
